# EUROPEAN PATENT SPECIFICATION

(11) **EP 3 494 993 B1**
(45) Date of publication and mention of the grant of the patent: **21.04.2021**
(21) Application number: 19153682.0
(22) Date of filing: 31.01.2012
(51) Int. Cl.: A61K 39/395, A61P 35/02, C12N 9/10, C12P 19/18, C12N 15/09, A23L 7/10, A23L 7/104, A23L 7/109, A21D 8/04, C07H 15/04

(54) **NOVEL USE OF MALTOTRIOSYL TRANSFERASE**
NEUE VERWENDUNG VON MALTOTRIOSYL-TRANSFERASE
NOUVELLE UTILISATION DE MALTOTRIOSYL TRANSFÉRASE

(30) Priority: 04.02.2011 JP 2011023281; 26.07.2011 JP 2011162973
(43) Date of publication of application: 12.06.2019
(62) Divisional of application: 12741768.1
(73) Proprietor: Amano Enzyme Inc., Nagoya-shi Aichi 460-8630 (JP)
(72) Inventor: OKADA, Masamichi, Kakamigahara-shi, Gifu 509-0109 (JP); YAMAGUCHI, Shotaro, Kakamigahara-shi, Gifu 509-0109 (JP)
(74) Representative: Witthoff Jaekel Steinecke Patentanwälte PartG mbB

(56) References cited:
- WO-A1-2011/001722
- JP-A- H07 236 496
- US-A1- 2010 256 345
- MOON Y H ET AL: "Synthesis, Structure Analyses, and Characterization of Novel Epigallocatechin Gallate (EGCG) Glycosides Using the Glucansucrase from Leuconostoc mesenteroides B-1299CB", JOURNAL OF AGRICULTURAL AND FOOD CHEMISTRY, AMERICAN CHEMICAL SOCIETY, US, vol. 54, no. 4, 22 February 2006 (2006-02-22), pages 1230-1237, XP002696285, ISSN: 0021-8561, DOI: 10.1021/JF052359I [retrieved on 2006-01-21]

## Description

### Technical Field

The present invention relates to a novel use of maltotriosyl transferase, and specifically to a method for producing a glycoside using maltotriosyl transferase.

### Background Art

Known examples of industrially used glycosyltransferases include α-glucosidase (production of isomaltooligosaccharide or nigerooligosaccharide), β-fructofuranosidase (production of fructooligosaccharide or lactosucrose), β-galactosidase (production of galactooligosaccharide), α-glucosyltransferase (production of palatinose), cyclodextrin glucanotransferase (production of cyclodextrin or coupling sugar), and branching enzymes (production of highly branched cyclic dextrin). Alfa-glucosidase and branching enzymes act on polysaccharides and oligosaccharides containing α-1,4 bonds to catalyze transglucosylation. Alfa-glucosidase catalyzes transglucosylation of monosaccharides, and branching enzymes catalyze transglucosylation of oligosaccharides of four or more sugars or polysaccharides.

One of uses of enzymes is the production of processed food containing starch. Starch aging causes considerable problems such as the deterioration of storage stability. Starch aging is mostly caused by aging of amylose molecules contained in starch, more specifically association and insolubilization of the amylose molecules (Non-patent Document 1). Therefore, control of starch aging through depolymerization of starch was studied, which allowed control of starch aging to some degree. However, depolymerization impairs the original properties of starch. Furthermore, under this method, decomposed starch has increased reducing power, and is stained upon reaction with protein or amino acid under heating. Therefore, this method has limited uses (Patent Document 1). Accordingly, aging control without depolymerization of starch has been studied.

Under the above circumstances, the present applicant reported a novel glycosyltransferase which catalyzes the transglucosylation of maltotriose units (hereinafter referred to as "maltotriosyl transferase" or "the present enzyme") and a method for producing the same in a preceding patent application, and showed the examples of the uses. (patent document 2: WO 2011/001722).

### Prior Art Documents

### Patent Document

Patent Document 1: Japanese Unexamined Patent Application Publication No. 2001-294601
Patent Document 2: WO 2011/001722
Patent Document 3: Japanese Unexamined Patent Application Publication No. 54-49354
Patent Document 4: Japanese Unexamined Patent Application Publication No. 2008-194024
Patent Document 5: Japanese Unexamined Patent Application Publication No. 9-107900
Patent Document 6: WO 2008/136331

### Non-patent Document

Non-patent Document 1: Okada et Al., Denpun Kagaku (Journal of the Japanese Society of Starch Science), 30(2), p. 223-230 (1983)

The production of kojic acid glucoside from a mixture of kojic acid and a glucan using an amylase in disclosed in JP 07236496.

### Summary of the Invention

### Problems to be Solved by the Invention

The present invention is intended to provide a novel use of maltotriosyl transferase found by the inventors. Said present invention is a method for producing a glycoside that is defined by the claims.

### Means for Solving the Problem

In consideration of the above-described problems, the inventors carried out various investigations, and focused attention on the production of glycosides. The present invention relates to a method for producing a glycoside, including a step of allowing maltotriosyl transferase to act on a donor substrate in the presence of a receptor substrate. In said method, the receptor substrate is kojic acid, ascorbic acid, hydroquinone, or glycerol.

In said method, the donor substrate is soluble starch, amylose, amylopectin, maltotetraose, maltopentaose, or maltohexaose.

In said method, the maltotriosyl transferase has the following enzymatic properties: the enzyme acts on polysaccharides and oligosaccharides having alpha-1,4 glucoside bonds to transfer maltotriose units to saccharides; when the enzyme acts on maltotetraose as the substrate, the ratio between the production rates of maltoheptaose and maltotriose is from 9:1 to 10:0 over the whole range of the substrate concentration from 0.67% (w/v) to 70% (w/v).

Said maltotriosyl transferase may be an enzyme derived from a microorganism.

Said maltotriosyl transferase may be an enzyme derived from Geobacillus species, such as *Geobacillus sp.* APC9669 (accession number NITE BP-770).

### Brief Description of the Drawings

FIG. 1 is a graph showing the optimal temperature of the maltotriosyl transferase derived from *Geobacillus sp.* APC9669.
FIG. 2 is a graph showing the optimal pH of the maltotriosyl transferase derived from *Geobacillus sp.* APC9669.
FIG. 3 is a graph showing the thermostability of the maltotriosyl transferase derived from *Geobacillus sp.* APC9669.
FIG. 4 is a graph showing the pH stability of the maltotriosyl transferase derived from *Geobacillus sp.* APC9669.
FIG. 5 shows the result of SDS-PAGE of the maltotriosyl transferase. Lane 1: molecular weight marker, and lane 2: maltotriosyl transferase.

### Description of Embodiments

### (Term)

In the present invention, the term "DNA coding a protein" refers to the DNA which gives the protein upon expression, more specifically the DNA having a base sequence corresponding to the amino acid sequence of the protein. Accordingly, codon degeneracy is taken into consideration.

In the present description, the term "isolated" may be replaced with "purified". When the enzyme of the present invention (maltotriosyl transferase) is derived from a natural material, the term "isolated" used for the enzyme means that the enzyme is substantially free of components of the natural material other than the enzyme (specifically substantially free of contaminant protein). Specifically, for example, in the isolated enzyme of the present invention, the content of contaminant proteins is less than about 20%, preferably less than about 10%, more preferably less than about 5%, and even more preferably less than about 1% in the weight equivalence. On the other hand, when the enzyme of the present invention is prepared by a genetic engineering technique, the term "isolated" means that the enzyme is substantially free of other components derived from the host cells or culture solution used. Specifically, for example, in the isolated enzyme of the present invention, the content of contaminant components is less than about 20%, preferably less than about 10%, more preferably less than about 5%, and even more preferably less than about 1% in the weight equivalence. In the present description, the simple term "maltotriosyl transferase" means "isolated maltotriosyl transferase", unless it is evident that the term has a different meaning. The same applies to the term "the present enzyme" used in place of maltotriosyl transferase.

The present invention preferably uses the maltotriosyl transferase (the present enzyme) disclosed by the inventors in the preceding patent application (see Patent Document 2 for more detail). As described in the preceding patent application, *Geobacillus sp.* APC9669 was found to produce maltotriosyl transferase, and the enzymatic properties were determined. The enzymatic properties thus determined are listed below.

### (1) Action

The present enzyme is a maltotriosyl transferase, and acts on polysaccharides and oligosaccharides having α-1,4 glucoside bonds as a binding mode to transfer maltotriose units to saccharides.

### (2) Substrate specificity

The present enzyme favorably acts on soluble starch, amylose, amylopectin, maltotetraose, maltopentaose, and maltohexaose. On the other hand, the present enzyme does not act on α-cyclodextrin, β-cyclodextrin, γ-cyclodextrin, maltotriose, and maltose.

### (3) Molecular weight

The molecular weight of the present enzyme is about 83,000 (by SDS-PAGE).

### (4) Optimum temperature

The optimum temperature for the present enzyme is about 50°C. The present enzyme exhibits high activity in the temperature range of about 45°C to 55°C. The optimum temperature was calculated by the below-described method for measuring maltotriosyl transferase activity (in 10 mmol/L MES buffer solution (pH 6.5)).

### (5) Optimum pH

The optimum pH for the present enzyme is about 7.5. The present enzyme exhibits high activity in the pH range of about 6.5 to 8.0. The optimum pH is determined based on, for example, the measurement in a universal buffer solution.

### (6) Thermostability

The present enzyme exhibits stable activity at 65°C or lower. The present enzyme keeps 90% or higher level of activity even after treatment for 30 minutes at 65°C in a 10 mmol/L MES buffer solution (pH 6.5).

### (7) pH stability

The present enzyme exhibits stable activity in a wide pH range of 5.0 to 10.0. More specifically, the enzyme keeps 85% or higher level of activity after treatment for 30 minutes at 40°C, as long as the pH of the enzyme solution used for the treatment is within the above range.

### (8) Isoelectric point

The isoelectric point of the present enzyme is about 4.5 (by Ampholine electrophoresis).

As shown in the prior patent application, when the maltotriosyl transferase produced by *Geobacillus sp.* APC9669 acts on maltotetraose as substrate, it gives a ratio between the maltoheptaose production rate and maltotriose production rate of 9:1 to 10:0 at any substrate concentration ranging from 0.67 to 70% (w/v), wherein maltoheptaose is a transglycosylation product and maltotriose is a decomposition product, respectively. In other words, the rate of transglucosylation is far higher over the wide substrate concentration range, and the maltoheptaose production rate was 90% or more, taking the sum of the maltoheptaose and maltotriose production rates as 100%. The rates were compared based on the molar ratios of the products.

The present enzyme is preferably a maltotriosyl transferase derived from *Geobacillus sp.* APC9669. The term "maltotriosyl transferase derived from *Geobacillus sp.* APC9669" in this case means a maltotriosyl transferase produced by *Geobacillus sp.* APC9669 (wild strain or mutant strain), or a maltotriosyl transferase obtained by a genetic engineering technique using the maltotriosyl transferase gene of *Geobacillus sp.* APC9669 (wild strain or mutant strain). Accordingly, "maltotriosyl transferase derived from *Geobacillus sp.* APC9669" includes the recombinants produced by host microorganisms into which the maltotriosyl transferase gene (or the modified version of the gene) obtained from *Geobacillus sp.* APC9669, *"Geobacillus sp.* APC9669 has been introduced.

For convenience of explanation, *Geobacillus sp.* APC9669 which is the source of the present enzyme is referred herein the bacterium producing the present enzyme. The APC9669 strain is deposited on the below-described depository, and is readily available therefrom.

Depository institution: Patent Microorganisms Depository, NITE Biotechnology Development Center (2-5-8, Kazusakamatari, Kisarazu-shi, Chiba, 292-0818, Japan)
Date of deposit (date of receipt): June 2, 2009
Accession number: NITE BP-770

After culturing the *Geobacillus sp.* APC9669, the present enzyme is collected from the culture solution and/or bacterial cells. The culture method may be liquid culture or solid culture, and preferably liquid culture. The conditions of liquid culture are described below.

The medium is not particularly limited as long as it is suitable for growing the microorganism used. Examples of the medium include those containing a carbon source such as glucose, sucrose, genthiobiose, soluble starch, glycerol, dextrin, molasses, or an organic acid, in addition, ammonium sulfate, ammonium carbonate, ammonium phosphate, ammonium acetate, or a nitrogen source such as peptone, yeast extract, corn steep liquor, casein hydrolysate, bran, or meat extract, in addition an inorganic salt such as a potassium salt, a magnesium salt, a sodium salt, a phosphate, a manganese salt, an iron salt, or a zinc salt. In order to accelerate the growth of the microorganism, a vitamin and an amino acid may be added to the medium. The pH of the medium is adjusted to, for example, about 3 to 10, and preferably about 7 to 8, and the incubation temperature is normally from about 10 to 80°C, preferably about 30 to 65°C. The microorganism is cultured for about 1 to 7 days, preferably for about 2 to 4 days under aerobic conditions. The culture method may be, for example, a shake culture method or an aerobic deep culture method using a jar fermenter.

After culturing under the above-described conditions, a maltotriosyl transferase is collected from the culture solution or bacterial cells. When the enzyme is collected from the culture solution, for example, the culture supernatant is subjected to, for example, filtration or centrifugation thereby removing insoluble matter, followed by separation and purification through an appropriate combination of, for example, concentration using an ultrafiltration membrane, salting out by ammonium sulfate precipitation, dialysis, and various chromatography procedures such as ion exchange chromatography, and thus obtaining the present enzyme.

On the other hand, when the enzyme is collected from bacterial cells, the bacterial cells are crushed by, for example, pressurization or ultrasonication, and then subjected to separation and purification in the same manner as described-above, and thus obtaining the present enzyme. Alternatively, the bacterial cells may be collected in advance from the culture solution by, for example, filtration or centrifugation, and then the above-described procedure (crushing of bacterial cells, separation, and purification) may be carried out.

Confirmation of expression and identification of the expression product are readily achieved using an antibody against the maltotriosyl transferase. Alternatively, the expression may be confirmed by measuring the maltotriosyl transferase activity.

The maltotriosyl transferase for the present invention according to one embodiment contains the amino acid sequence set forth in SEQ ID NO: 3. This amino acid sequence is the amino acid sequence set forth in SEQ ID NO: 2 excluding the signal peptide portion. The amino acid sequence set forth in SEQ ID NO: 2 was deduced from the base sequence (SEQ ID NO: 1) of the gene obtained by cloning *Geobacillus sp.* APC9669. In general, when the amino acid sequence of a certain protein is partially modified, the modified protein may have equivalent function to the unmodified protein. More specifically, modification of the amino acid sequence does not substantially influence the function of the protein, and thus the function of the protein may be maintained before and after the modification. Accordingly, for the present invention, use of a protein which is composed of an amino acid sequence equivalent to the amino acid sequence set forth in SEQ ID NO: 3, and has maltotriosyl transferase activity (hereinafter, also referred to as "equivalent protein") is also contemplated. The term "equivalent amino acid sequence" in this case means an amino acid sequence which is partially different from the amino acid sequence set forth in SEQ ID NO: 3, but the difference does not substantially influence the function of the protein (maltotriosyl transferase activity). The term "maltotriosyl transferase activity" means the activity for polysaccharides and oligosaccharides having α-1,4 glucoside bonds as a binding mode to transfer the maltotriose units to saccharides. The degree of the activity is not particularly limited as long as the function of a maltotriosyl transferase can be exhibited, but is preferably equivalent to or higher than that of the protein composed of the amino acid sequence set forth in SEQ ID NO: 3.

The term "partial difference in the amino acid sequence" typically means mutation (change) in the amino acid sequence caused by deletion or substitution of one to several (up to, for example, 3, 5, 7, or 10) amino acids composing the amino acid sequence, or addition, insertion, or combination thereof of one to several (up to, for example, 3, 5, 7, or 10) amino acids. The difference in the amino acid sequence is acceptable as long as the maltotriosyl transferase activity is maintained (the activity may be varied to a degree). As long as the conditions are satisfied, the position of the difference in the amino acid sequence is not particularly limited, and the difference may arise in a plurality of positions. The term "plurality" means, for example, a number corresponding to less than about 30%, preferably less than about 20%, more preferably less than about 10%, even more preferably less than about 5% of the total amino acids, and most preferably less than about 1%. More specifically, the equivalent protein has, for example, about 70% or more, preferably about 80% or more, even more preferably about 90% or more, even more preferably about 95% or more, and most preferably about 99% or more identity with the amino acid sequence set forth in SEQ ID NO: 3.

Preferably, the equivalence protein is obtained by causing conservative amino acid substitution in an amino acid residue which is not essential for maltotriosyl transferase activity. The term "conservative amino acid substitution" means the substitution of an amino acid residue with another amino acid residue having a side chain with similar properties. Amino acid residues are classified into several families according to their side chains, such as basic side chains (for example, ricin, arginine, and histidine), acidic side chains (for example, aspartic acid and glutamic acid), uncharged polar side chains (for example, glycine, asparagine, glutamine, serine, threonine, tyrosine, and cysteine), nonpolar side chains (for example, alanine, valine, leucine, isoleucine, proline, phenylalanine, methionine, and tryptophan), β branched side chains (for example, threonine, valine, and isoleucine), and aromatic side chains (for example, tyrosine, phenylalanine, tryptophan, and histidine). Conservative amino acid substitution is preferably the substitution between amino acid residues in one family.

The "equivalent protein" may have additional properties. For example, the equivalent protein may have higher stability than the protein composed of the amino acid sequence set forth in SEQ ID NO: 3, may perform a different function performed only at low temperatures and/or high temperatures, or may have a different optimum pH.

The identity (%) between the two amino acid sequences may be determined by, for example, the following procedure. Firstly, the two sequences are aligned for optimal comparison (for example, a gap may be introduced into the first sequence thereby optimizing the alignment with the second sequence). When the molecule at the specific position in the first sequence (amino acid residue) is the same as the molecule at the corresponding position in the second sequence, the molecules at the positions are regarded as identical. The sequence identity is the function of the number of the identical positions common to the two sequences (more specifically, identity (%) = number of identical positions / total number of positions × 100), and preferably the number and size of the gaps required for the optimization of the alignment are taken into consideration. Comparison of the two sequences and determination of the identity are achieved using a mathematic algorithm. Specific examples of the mathematic algorithm usable for the comparison of the sequences include, but not limited to, the algorithm described in Karlin and Altschul (1990) Proc. Natl. Acad. Sci. USA 87:2264-68, and modified in Karlin and Altschul (1993) Proc. Natl. Acad. Sci. USA 90:5873-77. These algorithms are incorporated into NBLAST Program and XBLAST Program (version 2.0) described in Altschul et al. (1990) J. Mol. Biol. 215:403-10. For example, under XBLAST Program, when BLAST polypeptide is searched under conditions that score = 50 and wordlength = 3, an amino acid sequence with a high identity can be obtained. Gapped BLAST described in Altschul et al. (1997), Amino Acids Research 25(17): 3389-3402 can be used for obtaining a gap alignment for comparison. When BLAST and Gapped BLAST are used, default parameters of corresponding programs (for example, XBLAST and NBLAST) may be used. For more detailed information, see, for example, the website of NCBI. Other examples of the mathematic algorithm usable for the comparison of the sequences include the algorithm described in Myersand Miller (1988) Comput Appl BioSci. 4: 11-17. These algorithms are incorporated into, for example, ALIGN Program usable in GENESTREAM Network Server (IGH Montpellier, France) or ISREC Server. When ALIGN program is used for the comparison of amino acid sequences, for example, a PAM120 weight residue table is used, wherein the gap length penalty is 12, and the gap penalty is 4. The identity between the two amino acid sequences is determined according to GAP Program of GCG software package using Blossom 62 matrix or PAM250 matrix, wherein the gap weight is 12, 10, 8, 6, or 4, and the gap length weight is = 2, 3, or 4.

The present enzyme may be a portion of a larger protein (for example, a fused protein). Examples of the sequence added to a fused protein include the sequences useful for purification of multiple histidine residues, and addition sequences which ensures stability in recombination production.

The present enzyme having the above-described amino acid sequence is readily prepared by a genetic engineering technique. For example, an appropriate host cell (for example, Escherichia coli) is transformed by a DNA coding the present enzyme (specific example is a DNA having nucleotide sequence of SEQ ID NO: 1), and the protein expressed in the transformant is collected, and thereby preparing the present enzyme. The collected protein is treated as appropriate according to the intended use. The present enzyme thus obtained as a recombinant protein may be subjected to various modifications. For example, the present enzyme composed of a recombinant protein linked to any peptide or protein can be obtained by producing a recombinant protein using a vector into which a DNA coding the present enzyme has been inserted together with other appropriate DNA. In addition, modification for causing addition of a sugar chain and/or a lipid, or N- or C-terminal processing may be carried out. These modifications allow, for example, extraction of a recombinant protein, simplification of purification, or addition of biological functions.

The method of the present invention may be used to provide a pharmaceutical composition, a quasi-drug composition, and a food composition.

The pharmaceutical composition and quasi-drug may be formulated according to a common procedure. The formulation may contain other pharmaceutically acceptable other components (for example, carrier, excipient, disintegrating agent, buffer, emulsifying agent, suspending agent, soothing agent, stabilizer, preservative, antiseptic, and normal saline solution). Examples of the excipient include lactose, starch, sorbitol, D-mannitol, and white sugar. Examples of the disintegrating agent include starch, carboxymethyl cellulose, and calcium carbonate. Examples of the buffer include phosphates, citrates, and acetates. Examples of the emulsifying agent include gum arabic, sodium alginate, and tragacanth. Examples of the suspending agent include glyceryl monostearate, aluminum monostearate, methyl cellulose, carboxymethyl cellulose, hydroxymethyl cellulose, and sodium lauryl sulfate. Examples of the soothing agent include benzyl alcohol, chlorobutanol, and sorbitol. Examples of the stabilizer include propylene glycol and ascorbic acid. Examples of the preservative include phenol, benzalkonium chloride, benzyl alcohol, chlorobutanol, and methylparaben. Examples of the antiseptic include benzalkonium chloride, paraoxybenzoic acid, and chlorobutanol.

The dosage form of the formulation is also not particularly limited. The pharmaceutical composition or quasi-drug composition may be provided in the form of, for example, pellets, powder, fine grains, granules, capsules, syrup, injection, external preparation, or suppository.

The pharmaceutical composition contains an active ingredient in an amount necessary to achieve the expected therapeutical or prophylactic benefit (more specifically therapeutically effective amount). In addition, the quasi-drug composition contains an active ingredient in an amount necessary to achieve the expected improving or prophylactic benefit. The amount of the active ingredient contained in the pharmaceutical composition or quasi-drug composition is, for example, about 0.1% by weight to about 95% by weight depending on the dosage form or shape, thereby achieving a desired dose.

Examples of the "food composition" include common food (grains, vegetable, meat, various processed food, confectionery, milk, refreshing beverage, alcohol beverage (for example, beer or beer-like beverage), food supplements (dietary supplements, nutrition drinks), food additives, pet food, and pet dietary supplements. Food supplements or food additives may be in the form of powder, granules, tablets, paste, or liquid. The form of a food composition makes it easy to routinely or continuously take the active ingredient of the present invention (indigestible saccharide). The food composition preferably contains the active ingredient in an amount effective to achieve therapeutical or prophylactic benefit. The loading may be established in consideration of, for example, the disease state, health condition, age, sex, body weight of the subject.

### (Use of maltotriosyl transferase in a: method for producing a glycoside)

The method of the present invention is to provide a method for producing a glycoside as a use of maltotriosyl transferase. In the method for producing a glycoside according to the present invention, in the same manner as the first aspect, preferably the maltotriosyl transferase (the present enzyme) disclosed by the applicants in the preceding patent application (see Patent Document 2 for more detail) is used. More specifically, the method for producing a glycoside according to the present invention includes a step of allowing maltotriosyl transferase (preferably the present enzyme) to act on a donor substrate in the presence of a receptor substrate.

According to the present invention, the receptor substrate include kojic acid, ascorbic acid, hydroquinone, and glycerol. Two or more receptor substrates may be used in combination. According to the present invention, the donor substrate include soluble starch, amylose, amylopectin, maltotetraose, maltopentaose, and maltohexaose. Two or more donor substrates may be used in combination. The loading of the present enzyme is not particularly limited, and may be from 1 U to 100 U for 1 g of the donor substrate. The temperature conditions during enzyme reaction are not particularly limited, and preferably from 30°C to 70°C, thereby allowing the sufficient action of the present enzyme.

### [Example]

### <Measurement method of maltotriosyl transferase activity>

The activity of the maltotriosyl transferase was measured as described below. More specifically, 0.5 mL of an enzyme solution was added to 2 mL of a 10 mmol/L MES buffer solution (pH 6.5) containing 1% maltotetraose (manufactured by Hayashibara Co., Ltd.), and allowed to stand at 40°C for 60 minutes. After standing, the mixture was heated for 5 minutes in a boiling water bath, and cooled in running water. The glucose thus formed was quantified using Glucose CII-Test Wako (manufactured by Wako Pure Chemical Industries, Ltd.). Under these conditions, the amount of the enzyme forming 1 µmol of glucose in 2.5 mL of the reaction solution for 1 minute was set at 1 unit.

### <Confirmation method of activity of maltotriosyl transferase>

In addition to the above-described <Measurement method of maltotriosyl transferase activity>, the activity of maltotriosyl transferase was confirmed as described below. More specifically, 15 µL of 1.0 u/mL enzyme solution was added to 985 µL of 5 mmol/L acetic acid buffer solution (pH 6.0) containing 10.3 mmol/L maltotetraose (manufactured by Hayashibara Co., Ltd.), and allowed to stand at 50°C for 1, 2, and 3 hours. After standing, the mixture was heated in a boiling water bath for 5 minutes, and then cooled in running water. The cooled reaction solution was desalted as needed using cationic and anionic resins, and the reaction solution was analyzed by HPLC. In the analysis, the HPLC apparatus was "Prominence UFLC" manufactured by Shimadzu Corporation, the column was "MCI GEL CK04S" manufactured by Mitsubishi Chemical Corporation, the eluent was water at a flow rate of 0.4 mL/minute, and the detector was a differential refractometer. The percentage of areas of the substrate and product thus obtained were converted into the molar quantity, and the consumption rate and production rate were calculated. For the purified maltotriosyl transferase, for example, the production rate ratio was heptaose : triose = about 92 : about 8.

As described in the preceding patent application (Patent Document 2), maltotriosyl transferase from *Geobacillus sp.* APC9669 was successfully obtained. The method for obtaining the enzyme and characteristics of the enzyme are cited from Patent Document 2 and described below.

### 1. Production and purification of maltotriosyl transferase from Geobacillus sp. APC9669

*Geobacillus sp.* APC9669 was cultured under shaking at 45°C for 2 days using a liquid culture medium having the components shown in Table 1. The culture supernatant thus obtained was concentrated five-fold using a UF membrane (AIP-1013D, manufactured by Asahi Kasei Corporation), and then ammonium sulfate was added to give a 50% saturated concentration. The precipitate fraction was dissolved again in 20 mmol/L tris-hydrochloride buffer solution (pH 8.0) containing 5 mmol/L calcium chloride, and subsequently ammonium sulfate was added to give a final concentration of 0.5 mol/L. The precipitate thus formed was removed by centrifugation, the remaining solution was passed through an HiLoad 26/10 Phenyl Sepharose HP column (manufactured by GE Healthcare) which had been equilibrated with 20 mmol/L tris-hydrochloride buffer solution (pH 8.0) containing 0.5 mol/L ammonium sulfate and 5 mmol/L calcium chloride, and the adsorbed maltotriosyl transferase protein was eluted by linear concentration gradient of ammonium sulfate from 0.5 mol/L to 0 mol/L.

**[Table 1]**

| Culture medium for producing maltotriosyl transferase | |
|---|---|
| | (w/v) |
| Yeast extract | 1.5% |
| Soybean peptone | 0.5% |
| Sodium chloride | 0.5% |
| Soluble starch | 0.4% |

The collected fraction containing active maltotriosyl transferase was concentrated using a UF membrane, and the buffer solution was replaced with a 20 mmol/L tris-hydrochloride buffer solution (pH 8.0) containing 5 mmol/L calcium chloride. The sample with a new buffer solution was passed through an HiLoad 26/10 Q Sepharose HP column (manufactured by GE Healthcare) which had been equilibrated with 20 mmol/L tris-hydrochloride buffer solution (pH 8.0) containing 5 mmol/L calcium chloride, and the adsorbed maltotriosyl transferase protein was eluted by linear concentration gradient of NaCl from 0 mol/L to 1 mol/L.

Furthermore, the collected fraction containing active maltotriosyl transferase was concentrated using a UF membrane, the buffer solution was replaced with a 50 mM phosphate buffer solution (pH 7.2) containing 0.15 M NaCl, and then the sample was passed through an HiLoad 26/60 Superdex 200 pg column (manufactured by GE Healthcare) which had been equilibrated with a 50 mM phosphate buffer solution (pH 7.2) containing 0.15 M NaCl, and eluted with the buffer. The fraction containing active maltotriosyl transferase was collected, desalted and concentrated using an ultrafiltration membrane, and thus a purified enzyme preparation was obtained. The purified enzyme thus obtained was subjected to the study of the following properties.

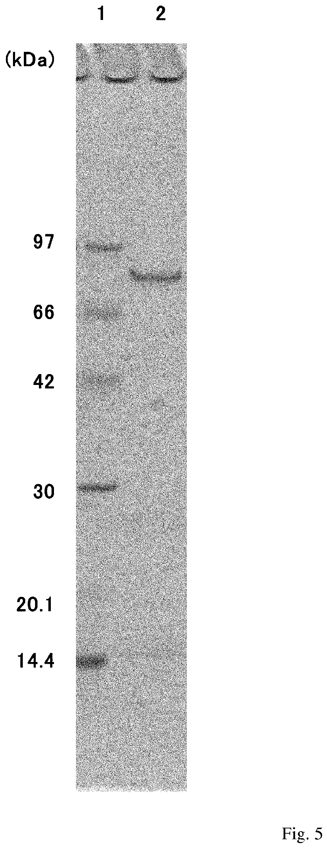

The results of the purification at each step are shown in Table 2. The specific activity at the final step was about 41 times in comparison with that of the crude enzyme. FIG. 5 shows the result of SDS-PAGE (CBB staining) on the samples at each step of purification using 10-20% gradient gel, indicating that the purified enzyme preparation (lane 2) is a single protein in SDS-PAGE.

**[Table 2]**

| | Total protein (mg) | Total activity (U) | Specific activity (u/mg) | Recovery (%) |
|---|---|---|---|---|
| Concentrate | 42 | 300 | 3.9 | 100 |
| Ammonium sulfate fraction | 8.5 | 220 | 22.9 | 73 |
| Phenyl HP | 0.50 | 58 | 100 | 19 |
| Q HP | 0.37 | 53 | 139 | 18 |
| Superdex 200 | 0.12 | 19 | 158 | 6.3 |

### 2. Properties of maltotriosyl transferase

### (1) Optimal reaction temperature

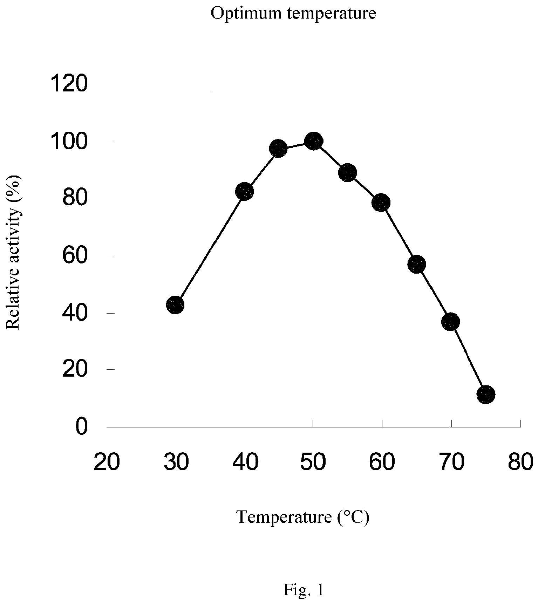

In the same manner as the above-described maltotriosyl transferase activity measurement method, the reaction was carried out at 30°C, 40°C, 45°C, 50°C, 55°C, 60°C, 65°C, 70°C, and 75°C. The results were expressed as relative activity, taking the value at the temperature which gave the maximum activity as 100%. The optimal reaction temperature was in the vicinity of 50°C (FIG. 1).

### (2) Optimal reaction pH

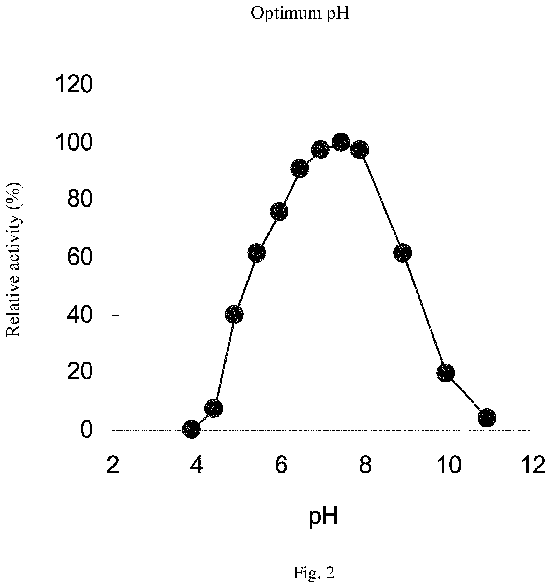

In the same manner as the above-described maltotriosyl transferase activity measurement method, the activity was measured under reaction conditions of 40°C and 60 minutes in buffers (universal buffer solution pH 4.0, pH 4.5, pH 5.0, pH 5.5, pH 6.0, pH 6.5, pH 7.0, pH 7.5, pH 8.0, pH 9.0, pH 10.0, and pH 11.0). The results were expressed as relative activity, taking the value at the pH which gave the maximum activity as 100%. The optimal reaction pH was in the vicinity of about 7.5 (FIG. 2).

### (3) Thermostability

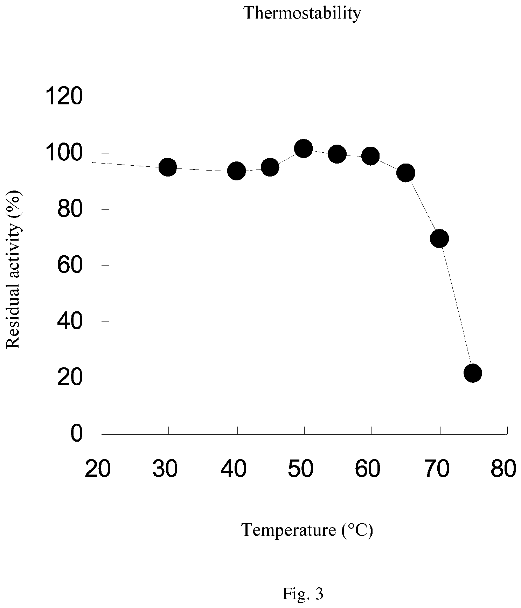

6 u/mL of enzyme solution was subjected to heat treatment in 10 mmol/L MES buffer solution (pH 6.5) for 30 minutes at 30°C, 40°C, 45°C, 50°C, 55°C, 60°C, 65°C, 70°C, and 75°C, and then the residual activity was measured by the above-described maltotriosyl transferase activity measurement method. The residual activity was expressed taking the activity untreated with heat as 100%. After heat treatment at 65°C for 30 minutes, residual activity was 90% or more, and stable up to 65°C (FIG. 3).

### (4) pH stability

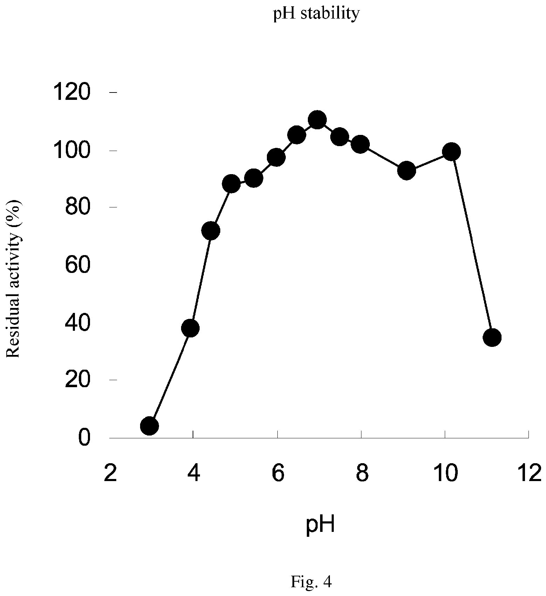

6 u/ mL of enzyme solution was treated at 40°C for 30 minutes in each buffer solution (universal buffer solution pH 3.0, pH 4.0, pH 4.5, pH 5.0, pH 5.5, pH 6.0, pH 6.5, pH 7.0, pH 7.5, pH 8.0, pH 9.0, pH 10.0, and pH 11.0), and the activity was measured by the above-described maltotriosyl transferase activity measurement method. In the range from pH 5.0 to pH 10.0, the residual activity was 85% or more, and stable from pH 5.0 to pH 10.0 (FIG. 4).

### (5) Molecular weight measurement by SDS-PAGE

SDS-PAGE was carried out in accordance with the method by Laemmli et al. The molecular weight marker used herein was Low Molecular Weight Calibration Kit for Electrophoresis (manufactured by GE Healthcare), which contained Phosphorylase b (97,000 Da), Albumin (66,000 Da), Ovalbumin (45,000 Da), Carbonic anhydrase (30,000 Da), Trypsin inhibitor (20,100 Da), and α-Lactalbumin (14,400 Da) as standard proteins. Electrophoresis was carried out for about 80 minutes at 20 mA/gel using a gradient gel (manufactured by Wako Pure Chemical Industries, Ltd.) having a gel concentration of 10-20%, and the molecular weight was determined; the molecular weight was about 83 kDa (FIG. 5).

### (6) Isoelectric point

The isoelectric point of the present enzyme was about 4.5 as measured by isoelectric focusing using ampholine (600V, 4°C, a current was passed for 48 hours).

### (7) Substrate specificity

The maltotriosyl transferase activity for different substrates was studied.

### a) Substrate specificity for maltooligosaccharide

Substrate specificity for maltooligosaccharide was studied as follows. The enzyme was added to each 10 mmol/L maltooligosaccharide to give a concentration of 0.002 u/mL, and allowed to stand at 50°C for 1, 2, and 3 hours. After standing, the mixture was heated for 5 minutes in a boiling water bath, and then cooled in running water. The cooled reaction solution was desalted as needed using cationic and anionic resins, and the reaction solution was analyzed by HPLC. The HPLC apparatus was "Prominence UFLC" manufactured by Shimadzu Corporation, the column was "MCI GEL CK04S" manufactured by Mitsubishi Chemical Corporation, the eluent was water at a flow rate of 0.4 mL/minute, and the detector was a differential refractometer. The percentage of areas of the substrate and product thus obtained were converted into the molar quantity, and the consumption rate and production rate were calculated. The rate of reaction for each maltooligosaccharide was calculated as follows. The rate for maltotetraose was the sum of the formation rate of heptaose and triose. The rate for maltopentaose was the sum of the production rate of octaose and triose. The rate for maltohexaose was calculated by halving the difference between the production rates of triose and nonose, and adding the value to the production rate of nonose.

**[Table 3]**

| Substrate | Relative rate (%) |
|---|---|
| Maltose | 0 |
| Maltotriose | 0 |
| Maltotetrao se | 83 |
| Maltopentaose | 100 |
| Maltohexaose | 89 |

For maltose and maltotriose, no reaction product was found. Sufficient action was observed on maltotetraose, maltopentaose, and maltohexaose.

### b) Substrate specificity for polysaccharide

For cyclodextrin, soluble starch, amylose, and amylopectin, the substrate specificity was studied by the following method. The enzyme was added at 0.002 u/mL with reference to 10 mmol/L of each maltooligosaccharide, and 0.1 u/mL of enzyme was allowed to stand at 50°C for 0, 1, 2, and 3 hours. After standing, the mixture was heated for 5 minutes in a boiling water bath, and cooled in running water. To 200 µL of the solution, Rhizopus-derived glucoamylase (manufactured by Wako Pure Chemical Industries, Ltd.) was added at 0.03 mg for 1.0 unit, and allowed to stand at 50°C overnight. After standing, the mixture was heated in a boiling water bath for 5 minutes, and then cooled in running water. The cooled reaction solution was desalted as needed using cationic and anionic resins, and the reaction solution was analyzed by HPLC. The HPLC apparatus was "Prominence UFLC" manufactured by Shimadzu Corporation, the column was "MCI GEL CK04S" manufactured by Mitsubishi Chemical Corporation, the eluent was water at a flow rate of 0.4 mL/minute, and the detector was a differential refractometer. In the comparison between the groups treated and untreated with the enzyme (maltotriosyl transferase), when the peaks of triose or higher saccharides increased with time, the product was judged as present (+), and when no increase was found, the product was judge as absent (-).

**[Table 4]**

| Substrate | Presence or absence of product |
|---|---|
| α-cyclodextrin | - |
| β-cyclodextrin | - |
| γ-cyclodextrin | - |
| Amylose | + |
| Amylopectin | + |
| Soluble starch | + |

For cyclodextrin, no reaction product was found. For soluble starch, amylose, and amylopectin, peaks of triose or higher saccharides increased with time. These polysaccharides were found to work as substrates. Since glucoamylase hydrolyzes α-1,4 and α-1,6 bonds, it was found that transglycosylation products were formed for other bonding patterns.

### (8) Influence of substrate concentration on enzyme reaction product

The influence of the substrate concentration on the enzyme reaction product was studied using maltotetraose as the substrate. The enzyme was added in such a manner that the residual amount of maltotetraose after reaction for 3 hours was 85% or more with reference to 0.67, 1.0, 3.0, 10, 30, and 70% (w/v) maltotetraose, and allowed to stand at 50°C for 1, 2, and 3 hours. After standing, the mixture was heated in a boiling water bath for 5 minutes, and cooled in running water. The cooled reaction solution was desalted as needed using cationic and anionic resins, and the reaction solution was analyzed by HPLC. The HPLC apparatus was "Prominence UFLC" manufactured by Shimadzu Corporation, the column was "MCI GEL CK04S" manufactured by Mitsubishi Chemical Corporation, the eluent was water at a flow rate of 0.4 mL/minute, and the detector was a differential refractometer. The percentage of areas of the substrate and product thus obtained were converted into the molar quantity, and the production rate was calculated. As a result of this, the rate of transglucosylation was 90% or more at all the substrate concentrations (0.67 to 70% (w/v)).

**[Table 5]**

| Substrate concentration (% (w/v)) | Reaction formation rate (molar ratio) | |
|---|---|---|
| | Transfer product (heptaose) | Decomposition product (triose) |
| 0.67 | 92% | 8% |
| 1.0 | 96% | 4% |
| 3.0 | 100% | 0% |
| 10 | 100% | 0% |
| 30 | 100% | 0% |
| 70 | 100% | 0% |

### 7. Production of hetero-oligosaccharide

Maltotetraose as the donor substrate and a receptor substrate (D-xylose, D-fructose, D-glucose, L-fucose, L-sorbose, D-mannose, D-sorbitol, D-galactose, N-acetylglucosamine, L-arabinose, D-ribose, or L-rhamnose) were dissolved in 10 mmol/L MES buffer solution (pH 6.5) to give a final concentration of 3.0% (w/v) or 10.0% (w/v). 1 u of maltotriosyl transferase was added to 1 mL of the substrate solution, and the mixture was allowed to stand and react in a water bath at 50°C for 24 hours. Subsequently, the reaction was stopped by boiling for 10 minutes. The reaction solution was subjected to HPLC analysis. In the HPLC analysis, the column was CK04S manufactured by Mitsubishi Chemical Corporation, the column temperature was 80°C, the mobile phase was ultrapure water at a flow rate of 0.4 mL/min, and the detector was a differential refractometer. The formation of the product was calculated, taking the formation of the receptor substrate before reaction as 100%. As shown in Table 9, various hetero-oligosaccharides were produced using various receptor substrates in the presence of maltotriosyl transferase. These hetero-oligosaccharides likely have various functions of commercially available functional oligosaccharides, such as anticarious action and intestine regulation action. Examples of the donor substrate other than maltotetraose include dextrin and starch.

**[Table 9]**

| Receptor substrate | Formation (%) |
|---|---|
| D-xylose | 26.3 |
| D-fructose | 17.0 |
| D-glucose | 15.9 |
| L-fucose | 12.9 |
| L-sorbose | 12.5 |
| D-mannose | 11.3 |
| D-sorbitol | 9.2 |
| D-galactose | 8.2 |
| N-acetylgluco samine | 8.1 |
| L-arabinose | 7.8 |
| D-ribose | 7.7 |
| L-rhamnose | 3.3 |

### 8. Production of glycoside 1

Amylose EX-1 as the donor substrate and a receptor substrate (kojic acid, ascorbic acid, or hydroquinone) were dissolved in 5 mmol/L MES buffer solution (pH 6.0) to give a final concentration of 2.5% (w/v). 1 u of maltotriosyl transferase was added to 1 mL of the substrate solution, and allowed to react in a water bath at 50°C for 24 hours. Subsequently, the reaction was stopped by boiling for 10 minutes. The reaction solution was subjected to HPLC analysis. In the HPLC analysis, the column was TSK gel ODS-100V 3 µm (4.6 mm×7.5 cm; manufactured by Tosoh Corporation), the column temperature was 50°C, the flow rate was 1.0 mL/min, and the detector was a UV detector. The mobile phase was composed of a solution A of 0.1% phosphoric acid and a solution B of methanol: ultrapure water = 7:3, and elution was carried out in the pattern of 0 minutes (B: 0%) → 4 minutes (B: 0%) → 8 minutes (B: 100%) → 10 minutes (B: 100% → B: 0%) → 15 minutes (B: 0%). The formation of the product was calculated, taking the molar amount of the receptor substrate before reaction as 100%. Various glycosides were produced by the use of maltotriosyl transferase. Sugar bonding by maltotriosyl transferase allows the production of a glycoside having improved functions and properties, such as improved solubility and stability of the substance.

**[Table 10]**

| Receptor substrate | Formation (%) |
|---|---|
| Kojic acid | 6.1 |
| Ascorbic acid | 1.9 |
| Hydroquinone | 1.5 |

### 9. Production of glycoside 2

Maltotetraose as the donor substrate and glycerol as the receptor substrate were dissolved in 10 mmol/L MES buffer solution (pH 6.5) to give a final concentration of 3.0% (w/v) or 10.0% (w/v). 1 u of maltotriosyl transferase was added to 1 mL of the substrate solution, and allowed to stand and react in a water bath at 50°C for 24 hours. Subsequently, the reaction was stopped by boiling for 10 minutes. The reaction solution was subjected to HPLC analysis. In the HPLC analysis, the column was CK04S (manufactured by Mitsubishi Chemical Corporation), the column temperature was 80°C, the mobile phase was ultrapure water at a flow rate of 0.4 mL/min, and the detector was a differential refractometer. The formation of the product was calculated, taking the amount of the receptor substrate before reaction as 100%. Through the use of maltotriosyl transferase, a glycerol glycoside was produced in a molar amount of 7.3% with reference to the receptor substrate. The glycerol glycoside is a non-reducing glycoside contained in sake mash, mirin (Japanese sweet rice wine for cooking), and miso, and is said to impart sharp sweetness and richness to Japanese sake. In addition, the degree of sweetness of the glycoside is half that of sugar, and the glycoside can be added to food and beverages as a sweetener which has a low browning potential, low Maillard reactivity, high thermal stability, anticarious effect, indigestibility, low hygroscopicity, and high moisture retentivity.

The present invention will not be limited to the above-described embodiments and examples of the present invention. The present invention includes various modifications which can be readily made by those skilled in the art without departing from the claims.

## Claims

1. A method for producing a glycoside, comprising a step of allowing maltotriosyl transferase to act on a donor substrate in the presence of a receptor substrate, wherein
(i) the receptor substrate is kojic acid, ascorbic acid, hydroquinone, or glycerol,
(ii) the donor substrate is soluble starch, amylose, amylopectin, maltotetraose, maltopentaose, or maltohexaose, and
(iii) the maltotriosyl transferase has the following enzymatic properties:
the enzyme acts on polysaccharides and oligosaccharides having α-1,4 glucoside bonds to transfer maltotriose units to saccharides; when the enzyme acts on maltotetraose as the substrate, the ratio between the production rates of maltoheptaose and maltotriose is from 9:1 to 10:0 over the whole range of the substrate concentration from 0.67% (w/v) to 70% (w/v).

2. The method for producing a glycoside according to claim 1, wherein the maltotriosyl transferase is an enzyme derived from a microorganism.

3. The method for producing a glycoside according to claim 1 or 2, wherein the maltotriosyl transferase is an enzyme derived from Geobacillus species.

4. The method for producing a glycoside according to claim 3, wherein the Geobacillus species is *Geobacillus sp.* APC9669 (accession number NITE BP-770).

5. The method for producing a glycoside according to any one of claims 1 to 4, wherein the maltotriosyl transferase
(a) has the following enzymatic properties:
(1) action: the enzyme acts on polysaccharides and oligosaccharides having α-1,4 glucoside bonds to transfer maltotriose units to saccharides;
(2) substrate specificity: the enzyme acts on soluble starch, amylose, amylopectin, maltotetraose, maltopentaose, and maltohexaose, but does not act on α-cyclodextrin, β-cyclodextrin, γ-cyclodextrin, maltotriose, and maltose; and
(3) molecular weight: about 83,000 (by SDS-PAGE), and/or
(b) is composed of an amino acid sequence SEQ ID NO. 3, an amino acid sequence which is 70% or more identical to the amino acid sequence SEQ ID NO.3 and shows maltotriosyl transferase activity, or a fragment of the amino acid sequence SEQ ID NO.3 showing maltotriosyl transferase activity.

## Patentansprüche

1. Verfahren zur Herstellung eines Glykosids, umfassend einen Schritt des Einwirkenlassens von Maltotriosyl-Transferase auf ein Donorsubstrat in Gegenwart eines Rezeptorsubstrats, wobei
(i) das Rezeptorsubstrat Kojisäure, Ascorbinsäure, Hydrochinon oder Glycerin ist,
(ii) das Donorsubstrat lösliche Stärke, Amylose, Amylopektin, Maltotetraose, Maltopentaose oder Maltohexaose ist, und
(iii) die Maltotriosyl-Transferase die folgenden enzymatischen Eigenschaften hat:
das Enzym wirkt auf Polysaccharide und Oligosaccharide mit α-1,4-Glucosidbindungen, um Maltotrioseeinheiten auf Saccharide zu übertragen; wenn das Enzym auf Maltotetraose als Substrat wirkt, ist das Verhältnis zwischen den Produktionsraten von Maltoheptaose und Maltotriose von 9:1 bis 10:0 über den gesamten Bereich der Substratkonzentration von 0,67% (w/v) bis 70% (w/v).

2. Verfahren zur Herstellung eines Glykosids nach Anspruch 1, wobei die Maltotriosyl-Transferase ein Enzym ist, das von einem Mikroorganismus stammt.

3. Verfahren zur Herstellung eines Glykosids nach Anspruch 1 oder 2, wobei die Maltotriosyl-Transferase ein Enzym ist, das von Geobacillus-Spezies stammt.

4. Verfahren zur Herstellung eines Glykosids nach Anspruch 3, wobei die Geobacillus-Spezies *Geobacillus sp.* APC9669 (Hinterlegungsnummer NITE BP-770) ist.

5. Verfahren zur Herstellung eines Glykosids nach einem der Ansprüche 1 bis 4, wobei die Maltotriosyl-Transferase
(a) die folgenden enzymatischen Eigenschaften aufweist:
(1) Wirkung: das Enzym wirkt auf Polysaccharide und Oligosaccharide mit α-1,4-Glucosidbindungen, um Maltotrioseeinheiten auf Saccharide zu übertragen;
(2) Substratspezifität: das Enzym wirkt auf lösliche Stärke, Amylose, Amylopektin, Maltotetraose, Maltopentaose und Maltohexaose, wirkt aber nicht auf α-Cyclodextrin, β-Cyclodextrin, γ-Cyclodextrin, Maltotriose und Maltose; und
(3) Molekulargewicht: etwa 83.000 (durch SDS-PAGE), und/oder
(b) aus einer Aminosäuresequenz SEQ ID NO. 3, einer Aminosäuresequenz, die zu 70% oder mehr mit der Aminosäuresequenz SEQ ID NO. 3 identisch ist und Maltotriosyl-Transferase-Aktivität zeigt, oder aus einem Fragment der Aminosäuresequenz SEQ ID NO. 3, das Maltotriosyl-Transferase-Aktivität zeigt, besteht.

## Revendications

1. Procédé pour produire un glycoside, comprenant une étape de permettre à la maltotriosyl transférase d'agir sur un substrat donneur en présence d'un substrat récepteur, où
(i) le substrat récepteur est l'acide kojique, l'acide ascorbique, l'hydroquinone ou le glycérol,
(ii) le substrat donneur est l'amidon soluble, l'amylose, l'amylopectine, le maltotétraose, le maltopentaose ou le maltohexaose, et
(iii) la maltotriosyl transférase a les propriétés enzymatiques suivantes:
l'enzyme agit sur les polysaccharides et les oligosaccharides ayant des liaisons α-1,4 glycosidiques pour transférer des unités maltotriose à des saccharides; lorsque l'enzyme agit sur le maltotétraose comme substrat, le rapport entre les taux de production de maltoheptaose et de maltotriose est de 9:1 à 10:0 sur toute la plage des concentrations de substrat de 0,67% (p/v) à 70 % (p/v).

2. Procédé pour produire un glycoside selon la revendication 1, où la maltotriosyl transférase est une enzyme dérivée d'un microorganisme.

3. Procédé pour produire un glycoside selon la revendication 1 ou 2, où la maltotriosyl transférase est une enzyme dérivée d'une espèce de Geobacillus.

4. Procédé pour produire un glycoside selon la revendication 3, où l'espèce de Geobacillus est *Geobacillus sp.* APC9669 (numéro d'accès NITE BP-770).

5. Procédé pour produire un glycoside selon l'une quelconque des revendications 1 à 4, où la maltotriosyl transférase
(a) a les propriétés enzymatiques suivantes:
(1) action: l'enzyme agit sur les polysaccharides et les oligosaccharides ayant des liaisons α-1,4 glycosidiques pour transférer des unités maltotriose à des saccharides;
(2) spécificité de substrat: l'enzyme agit sur l'amidon soluble, l'amylose, l'amylopectine, le maltotétraose, le maltopentaose et le maltohexaose, mais n'agit pas sur l'α-cyclodextrine, la β-cyclodextrine, la γ-cyclodextrine, le maltotriose et le maltose; et
(3) poids moléculaire: environ 83000 (par SDS-PAGE), et/ou
(b) est composée d'une séquence d'acides aminés SEQ ID NO. 3, d'une séquence d'acides aminés qui est identique à 70% ou plus à la séquence d'acides aminés SEQ ID NO. 3 et présente une activité maltotriosyl transférase, ou d'un fragment de la séquence d'acides aminés SEQ ID NO. 3 présentant une activité maltotriosyl transférase.


### SEQUENCE LISTING

<110> AMANO ENZYME INC.
   OKADA, Masamichi
   YAMAGUCHI, Shotaro
<120> NOVEL USE OF MALTOTRIOSYL TRANSFERASE
<130> AE11001P
<150> JP P2011-023281
   <151> 2011-02-04
<150> JP P2011-162973
   <151> 2011-07-26
<160> 3
<170> PatentIn version 3.5
<210> 1
   <211> 2304
   <212> DNA
   <213> Geobacillus sp.
<400> 1
   atgtggaagg ttccaaaatt cattaaacaa tcatatctcg tttttcttct tgctctattg      60
   ctctattctt catttggttt ttcgttttcc aggactgagg cgactacatc tacaggagct     120
   ttaggaccag ttaccccgaa agacacaata tatcagatcg tgacggaccg tttttttgat     180
   ggcgacccat caaataacaa gcctcctggt tttgatccta ccctgtttga tgatccggac     240
   ggcaataacc aggggaacgg aaaagattta aagttgtatc aaggcggtga tttccaagga     300
   atcatagata aaattcctta tttaaaaaat atggggataa ctgccgtttg gatttccgct     360
   ccttatgaaa atagggacac tgtgatagaa gattatcaat cagatggaag tattaatcgt     420
   tggaccagtt tccacggtta ccatgcaaga aattattttg caactaacaa acattttggt     480
   actatgaaag attttattag actacgcgat gctttgcatc aaaatggaat taaactagtg     540
   atagattttg tatccaatca ttcgagtcgt tggcaaaacc cgacattgaa ttttgcgcct     600
   gaagatggta aattatatga acctgacaag gatgcgaatg gcaattacgt atttgatgct     660
   aatggagagc ctgcggatta caacggtgat ggaaaagttg agaatctcct ggcggatcca     720
   cataacgacg tgaatggttt tttccatggt ctgggtgacc ggggcaacga tacttctcgt     780
   tttggctacc gttacaaaga tctaggttct ttggctgatt attctcagga aaatgcacta     840
   gtggttgaac atttggagaa agcagctaaa ttttggaaat caaaagggat cgatggtttt     900
   cgacatgatg ccactttgca tatgaatcct gcatttgtga agggatttaa agatgcaatt     960
   gattcagatg caggtggccc ggttacccat tttggtgaat ttttcattgg aagaccggat    1020
   cccaagtatg atgagtaccg gacatttcct gaacgaacag gagtcaacaa cttggatttt    1080
   gaatatttcc gtgcggccac aaacgcattt gggaactttt ctgaaacgat gagttccttt    1140
   ggtgatatga tgatcaagac aagtaatgat tacatttatg aaaatcaaac agttactttc 1200
   ttggataatc atgatgtaac aagatttcgc tatattcaac caaacgataa accttatcat 1260
   gcagctctcg ctgtcttgat gacatcacgt gggattccta acatttatta cggaacagag 1320
   caatatctga tgccgtcaga ctcaagtgac attgcgggtc ggatgtttat gcagacttct 1380
   actaacttcg atgaaaatac cactgcatat aaagtcattc aaaagctttc aaacttaaga 1440
   aaaaataatg aagctattgc ctatggaacc acagaaattt tatacagcac aaatgatgta 1500
   ctggtcttta aaagacagtt ctatgataaa caagtaattg tagcggtaaa ccgacaaccg 1560
   gatcaaacgt ttaccattcc ggagttagat acgactcttc cagtaggaac ctatagtgat 1620
   gtactgggtg gactgttata tgggagttca atgagcgtaa ataatgtcaa cggtcaaaac 1680
   aaaatttcta gctttacctt gtctggagga gaggtcaatg tctggtcgta taacccatca 1740
   ttggggactt taactccaag gattggcgac gttatttcca ccatgggacg tcccggtaat 1800
   accgtttaca tttacggtac tggattagga ggaagcgtaa cagtcaaatt cggttctact 1860
   gttgctactg tggtgtcaaa cagcgatcaa atgattgagg ctatagttcc aaacactaat 1920
   cctggaattc aaaatattac agttacaaaa ggatctgtaa ccagtgatcc tttccgatat 1980
   gaggtcctat ccggcgatca ggtgcaagta atttttcatg tgaatgccac aacgaattgg 2040
   ggggaaaaca tttatgttgt cggaaacatt ccagagttgg gaagctggga tccgaaccaa 2100
   tcgtctgagg cgatgttaaa tccgaactat ccagaatggt tcttgccagt gagtgtgccc 2160
   aagggagcta cttttgaatt caagtttatc aaaaaagata acaatggaaa tgtcatttgg 2220
   gaaagcagga gcaacagagt atttaccgca ccgaacagtt cgaccggtac tattgacacc 2280
   cctttatatt tttgggataa ctaa 2304
<210> 2
   <211> 767
   <212> PRT
   <213> Geobacillus sp.
<400> 2
   Met Trp Lys Val Pro Lys Phe Ile Lys Gln Ser Tyr Leu Val Phe Leu
   1               5                   10                  15
   Leu Ala Leu Leu Leu Tyr Ser Ser Phe Gly Phe Ser Phe Ser Arg Thr
               20                  25                  30
   Glu Ala Thr Thr Ser Thr Gly Ala Leu Gly Pro Val Thr Pro Lys Asp
           35                  40                  45
   Thr Ile Tyr Gln Ile Val Thr Asp Arg Phe Phe Asp Gly Asp Pro Ser
       50                  55                  60
   Asn Asn Lys Pro Pro Gly Phe Asp Pro Thr Leu Phe Asp Asp Pro Asp
   65                  70                  75                  80
   Gly Asn Asn Gln Gly Asn Gly Lys Asp Leu Lys Leu Tyr Gln Gly Gly
                   85                  90                  95
   Asp Phe Gln Gly Ile Ile Asp Lys Ile Pro Tyr Leu Lys Asn Met Gly
               100                 105                 110
   Ile Thr Ala Val Trp Ile Ser Ala Pro Tyr Glu Asn Arg Asp Thr Val
           115                 120                 125
   Ile Glu Asp Tyr Gln Ser Asp Gly Ser Ile Asn Arg Trp Thr Ser Phe
       130                 135                 140
   His Gly Tyr His Ala Arg Asn Tyr Phe Ala Thr Asn Lys His Phe Gly
   145                 150                 155                 160
   Thr Met Lys Asp Phe Ile Arg Leu Arg Asp Ala Leu His Gln Asn Gly
                   165                 170                 175
   Ile Lys Leu Val Ile Asp Phe Val Ser Asn His Ser Ser Arg Trp Gln
               180                 185                 190
   Asn Pro Thr Leu Asn Phe Ala Pro Glu Asp Gly Lys Leu Tyr Glu Pro
           195                 200                 205
   Asp Lys Asp Ala Asn Gly Asn Tyr Val Phe Asp Ala Asn Gly Glu Pro
       210                 215                 220
   Ala Asp Tyr Asn Gly Asp Gly Lys Val Glu Asn Leu Leu Ala Asp Pro
   225                 230                 235                 240
   His Asn Asp Val Asn Gly Phe Phe His Gly Leu Gly Asp Arg Gly Asn
                   245                 250                 255
   Asp Thr Ser Arg Phe Gly Tyr Arg Tyr Lys Asp Leu Gly Ser Leu Ala
               260                 265                 270
   Asp Tyr Ser Gln Glu Asn Ala Leu Val Val Glu His Leu Glu Lys Ala
           275                 280                 285
   Ala Lys Phe Trp Lys Ser Lys Gly Ile Asp Gly Phe Arg His Asp Ala
       290                 295                 300
   Thr Leu His Met Asn Pro Ala Phe Val Lys Gly Phe Lys Asp Ala Ile
   305 310 315 320
   Asp Ser Asp Ala Gly Gly Pro Val Thr His Phe Gly Glu Phe Phe Ile
   325 330 335
   Gly Arg Pro Asp Pro Lys Tyr Asp Glu Tyr Arg Thr Phe Pro Glu Arg
   340 345 350
   Thr Gly Val Asn Asn Leu Asp Phe Glu Tyr Phe Arg Ala Ala Thr Asn
   355 360 365
   Ala Phe Gly Asn Phe Ser Glu Thr Met Ser Ser Phe Gly Asp Met Met
   370 375 380
   Ile Lys Thr Ser Asn Asp Tyr Ile Tyr Glu Asn Gln Thr Val Thr Phe
   385 390 395 400
   Leu Asp Asn His Asp Val Thr Arg Phe Arg Tyr Ile Gln Pro Asn Asp
   405 410 415
   Lys Pro Tyr His Ala Ala Leu Ala Val Leu Met Thr Ser Arg Gly Ile
   420 425 430
   Pro Asn Ile Tyr Tyr Gly Thr Glu Gln Tyr Leu Met Pro Ser Asp Ser
   435 440 445
   Ser Asp Ile Ala Gly Arg Met Phe Met Gln Thr Ser Thr Asn Phe Asp
   450 455 460
   Glu Asn Thr Thr Ala Tyr Lys Val Ile Gln Lys Leu Ser Asn Leu Arg
   465 470 475 480
   Lys Asn Asn Glu Ala Ile Ala Tyr Gly Thr Thr Glu Ile Leu Tyr Ser
   485 490 495
   Thr Asn Asp Val Leu Val Phe Lys Arg Gln Phe Tyr Asp Lys Gln Val
   500 505 510
   Ile Val Ala Val Asn Arg Gln Pro Asp Gln Thr Phe Thr Ile Pro Glu
   515 520 525
   Leu Asp Thr Thr Leu Pro Val Gly Thr Tyr Ser Asp Val Leu Gly Gly
   530 535 540
   Leu Leu Tyr Gly Ser Ser Met Ser Val Asn Asn Val Asn Gly Gln Asn
   545 550 555 560
   Lys Ile Ser Ser Phe Thr Leu Ser Gly Gly Glu Val Asn Val Trp Ser
   565 570 575
   Tyr Asn Pro Ser Leu Gly Thr Leu Thr Pro Arg Ile Gly Asp Val Ile
   580 585 590
   Ser Thr Met Gly Arg Pro Gly Asn Thr Val Tyr Ile Tyr Gly Thr Gly
   595 600 605
   Leu Gly Gly Ser Val Thr Val Lys Phe Gly Ser Thr Val Ala Thr Val
   610 615 620
   Val Ser Asn Ser Asp Gln Met Ile Glu Ala Ile Val Pro Asn Thr Asn
   625 630 635 640
   Pro Gly Ile Gln Asn Ile Thr Val Thr Lys Gly Ser Val Thr Ser Asp
   645 650 655
   Pro Phe Arg Tyr Glu Val Leu Ser Gly Asp Gln Val Gln Val Ile Phe
   660 665 670
   His Val Asn Ala Thr Thr Asn Trp Gly Glu Asn Ile Tyr Val Val Gly
   675 680 685
   Asn Ile Pro Glu Leu Gly Ser Trp Asp Pro Asn Gln Ser Ser Glu Ala
   690 695 700
   Met Leu Asn Pro Asn Tyr Pro Glu Trp Phe Leu Pro Val Ser Val Pro
   705 710 715 720
   Lys Gly Ala Thr Phe Glu Phe Lys Phe Ile Lys Lys Asp Asn Asn Gly
   725 730 735
   Asn Val Ile Trp Glu Ser Arg Ser Asn Arg Val Phe Thr Ala Pro Asn
   740 745 750
   Ser Ser Thr Gly Thr Ile Asp Thr Pro Leu Tyr Phe Trp Asp Asn
   755 760 765
<210> 3
   <211> 733
   <212> PRT
   <213> Geobacillus sp.
<400> 3
   Thr Thr Ser Thr Gly Ala Leu Gly Pro Val Thr Pro Lys Asp Thr Ile
   1 5 10 15
   Tyr Gln Ile Val Thr Asp Arg Phe Phe Asp Gly Asp Pro Ser Asn Asn
               20                  25                  30
   Lys Pro Pro Gly Phe Asp Pro Thr Leu Phe Asp Asp Pro Asp Gly Asn
           35                  40                  45
   Asn Gln Gly Asn Gly Lys Asp Leu Lys Leu Tyr Gln Gly Gly Asp Phe
       50                  55                  60
   Gln Gly Ile Ile Asp Lys Ile Pro Tyr Leu Lys Asn Met Gly Ile Thr
   65                  70                  75                  80
   Ala Val Trp Ile Ser Ala Pro Tyr Glu Asn Arg Asp Thr Val Ile Glu
                   85                  90                  95
   Asp Tyr Gln Ser Asp Gly Ser Ile Asn Arg Trp Thr Ser Phe His Gly
               100                 105                 110
   Tyr His Ala Arg Asn Tyr Phe Ala Thr Asn Lys His Phe Gly Thr Met
           115                 120                 125
   Lys Asp Phe Ile Arg Leu Arg Asp Ala Leu His Gln Asn Gly Ile Lys
       130                 135                 140
   Leu Val Ile Asp Phe Val Ser Asn His Ser Ser Arg Trp Gln Asn Pro
   145                 150                 155                 160
   Thr Leu Asn Phe Ala Pro Glu Asp Gly Lys Leu Tyr Glu Pro Asp Lys
                   165                 170                 175
   Asp Ala Asn Gly Asn Tyr Val Phe Asp Ala Asn Gly Glu Pro Ala Asp
               180                 185                 190
   Tyr Asn Gly Asp Gly Lys Val Glu Asn Leu Leu Ala Asp Pro His Asn
           195                 200                 205
   Asp Val Asn Gly Phe Phe His Gly Leu Gly Asp Arg Gly Asn Asp Thr
       210                 215                 220
   Ser Arg Phe Gly Tyr Arg Tyr Lys Asp Leu Gly Ser Leu Ala Asp Tyr
   225                 230                 235                 240
   Ser Gln Glu Asn Ala Leu Val Val Glu His Leu Glu Lys Ala Ala Lys
                   245                 250                 255
   Phe Trp Lys Ser Lys Gly Ile Asp Gly Phe Arg His Asp Ala Thr Leu
               260                 265                 270
   His Met Asn Pro Ala Phe Val Lys Gly Phe Lys Asp Ala Ile Asp Ser
           275                 280                 285
   Asp Ala Gly Gly Pro Val Thr His Phe Gly Glu Phe Phe Ile Gly Arg
       290                 295                 300
   Pro Asp Pro Lys Tyr Asp Glu Tyr Arg Thr Phe Pro Glu Arg Thr Gly
   305                 310                 315                 320
   Val Asn Asn Leu Asp Phe Glu Tyr Phe Arg Ala Ala Thr Asn Ala Phe
                   325                 330                 335
   Gly Asn Phe Ser Glu Thr Met Ser Ser Phe Gly Asp Met Met Ile Lys
               340                 345                 350
   Thr Ser Asn Asp Tyr Ile Tyr Glu Asn Gln Thr Val Thr Phe Leu Asp
           355                 360                 365
   Asn His Asp Val Thr Arg Phe Arg Tyr Ile Gln Pro Asn Asp Lys Pro
       370                 375                 380
   Tyr His Ala Ala Leu Ala Val Leu Met Thr Ser Arg Gly Ile Pro Asn
   385                 390                 395                 400
   Ile Tyr Tyr Gly Thr Glu Gln Tyr Leu Met Pro Ser Asp Ser Ser Asp
                   405                 410                 415
   Ile Ala Gly Arg Met Phe Met Gln Thr Ser Thr Asn Phe Asp Glu Asn
               420                 425                 430
   Thr Thr Ala Tyr Lys Val Ile Gln Lys Leu Ser Asn Leu Arg Lys Asn
           435                 440                 445
   Asn Glu Ala Ile Ala Tyr Gly Thr Thr Glu Ile Leu Tyr Ser Thr Asn
       450                 455                 460
   Asp Val Leu Val Phe Lys Arg Gln Phe Tyr Asp Lys Gln Val Ile Val
   465                 470                 475                 480
   Ala Val Asn Arg Gln Pro Asp Gln Thr Phe Thr Ile Pro Glu Leu Asp
                   485                 490                 495
   Thr Thr Leu Pro Val Gly Thr Tyr Ser Asp Val Leu Gly Gly Leu Leu
               500                 505                 510
   Tyr Gly Ser Ser Met Ser Val Asn Asn Val Asn Gly Gln Asn Lys Ile
           515                 520                 525
   Ser Ser Phe Thr Leu Ser Gly Gly Glu Val Asn Val Trp Ser Tyr Asn
       530                 535                 540
   Pro Ser Leu Gly Thr Leu Thr Pro Arg Ile Gly Asp Val Ile Ser Thr
   545                 550                 555                 560
   Met Gly Arg Pro Gly Asn Thr Val Tyr Ile Tyr Gly Thr Gly Leu Gly
                   565                 570                 575
   Gly Ser Val Thr Val Lys Phe Gly Ser Thr Val Ala Thr Val Val Ser
               580                 585                 590
   Asn Ser Asp Gln Met Ile Glu Ala Ile Val Pro Asn Thr Asn Pro Gly
           595                 600                 605
   Ile Gln Asn Ile Thr Val Thr Lys Gly Ser Val Thr Ser Asp Pro Phe
       610                 615                 620
   Arg Tyr Glu Val Leu Ser Gly Asp Gln Val Gln Val Ile Phe His Val
   625                 630                 635                 640
   Asn Ala Thr Thr Asn Trp Gly Glu Asn Ile Tyr Val Val Gly Asn Ile
                   645                 650                 655
   Pro Glu Leu Gly Ser Trp Asp Pro Asn Gln Ser Ser Glu Ala Met Leu
               660                 665                 670
   Asn Pro Asn Tyr Pro Glu Trp Phe Leu Pro Val Ser Val Pro Lys Gly
           675                 680                 685
   Ala Thr Phe Glu Phe Lys Phe Ile Lys Lys Asp Asn Asn Gly Asn Val
       690                 695                 700
   Ile Trp Glu Ser Arg Ser Asn Arg Val Phe Thr Ala Pro Asn Ser Ser
   705                 710                 715                 720
   Thr Gly Thr Ile Asp Thr Pro Leu Tyr Phe Trp Asp Asn
                   725                 730